Europäisches Patentamt
European Patent Office
Office européen des brevets

(11) Numéro de publication: **0 098 006**
**A2**

(12) # DEMANDE DE BREVET EUROPEEN

(21) Numéro de dépôt: 83200906.2

(22) Date de dépôt: 20.06.83

(51) Int. Cl.[3]: C 07 D 251/34
C 07 D 273/04

(30) Priorité: 29.06.82 LU 84241

(43) Date de publication de la demande: 11.01.84 Bulletin 84/2

(84) Etats contractants désignés: BE CH DE FR GB IT LI NL

(71) Demandeur: SOCIETE CARBOCHIMIQUE en abrégé "CARBOCHIM"
avenue de la Renaissance 12
B-1040 Bruxelles(BE)

(72) Inventeur: Lambert, Pierre
Rue de la Citronnelle, 19A
B-1348 Louvain-la-Neuve(BE)

(72) Inventeur: De Aguirre-Otegui, Ignacio
Chemin des Maréchaux, 7
B-1350 Wavre-Limal(BE)

(74) Mandataire: De Brabanter, Maurice et al,
Bureau VANDER HAEGHEN 63 Avenue de la Toison d'Or
B-1060 Bruxelles(BE)

(54) **Isocyanurates et leur préparation.**

(57) L'invention concerne de nouveaux isocyanurates ou triazinates 1,3,5 triones 2,4,6 et leur préparation par réaction d'une oxadiazine 1,3,5 trione 2,4,6 avec un cyanate salin, tel qu'un cyanate d'un métal, d'ammonium, de phosphonium ou d'arsonium.

L'invention concerne aussi des triazines 1,3,5 triones 2,4,6 moléculaires préparées par réaction d'un isocyanurate ou triazinate 1,3,5 trione 2,4,6 avec un acide minéral ou organique ou avec un halogénure ou un sulfate organique.

EP 0 098 006 A2

ISOCYANURATES ET LEUR PREPARATION

La présente invention est relative à de nouveaux isocyanurates appelés également triazinates 1,3,5 triones 2,4,6.

Les nouveaux isocyanurates suivant l'invention répondent à la formule générale :

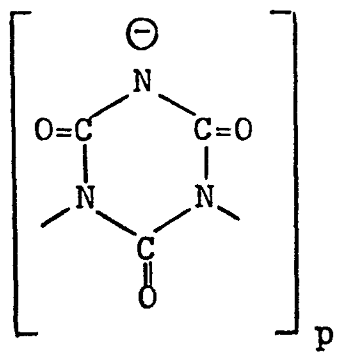

$$(R)_a\ (R')_b\ \left[Q^{n\oplus}\right]_{p/n}$$

(I)

dans laquelle

R désigne un radical hydrocarboné éventuellement substitué, monovalent ou polyvalent ;

R' qui est différent de R désigne un radical hydrocarboné éventuellement substitué ou de l'hydrogène ;

Q désigne un groupement contenant un nombre n de fonctions ammonium, phosphonium ou arsonium tétrasubstituées ;

p est un nombre au moins égal à 1 ;

a est égal à 1 ou à p et b est égal à zéro ou à p, étant entendu que, si a est égal à 1, b est égal à p et que, si b est égal à zéro, a est égal à p.

L'invention couvre, en particulier à titre de nouveaux isocyanurates, les composés répondant aux formules suivantes :

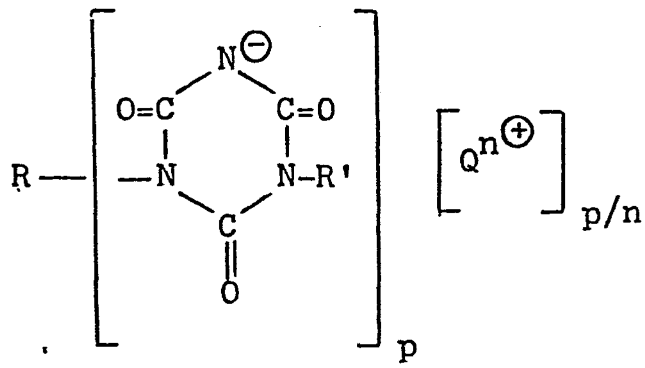

(II)

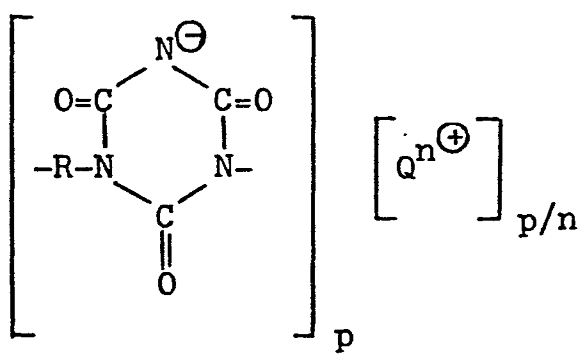

(III)

dans lesquelles

Q désigne un groupement contenant un nombre n de fonctions ammonium, phosphonium ou arsonium tétrasubstituées ;

R désigne un radical hydrocarboné éventuellement substitué, monovalent ou polyvalent ;

R' désigne un radical hydrocarboné éventuellement substitué, différent de R, ou de l'hydrogène ;

p est un nombre égal ou supérieur à 1 ;

n désigne le nombre de fonctions ammonium, phosphonium ou arsonium présentes dans le groupement Q.

Les isocyanurates de formules (I), (II) et (III) suivant l'invention peuvent être transformés, de manière connue, en isocyanurates moléculaires mono-, bi- ou trisubstitués dont tous les substituants peuvent être identiques ou différents l'un de l'autre.

Les nouveaux isocyanurates de formules (I), (II) et (III) suivant l'invention peuvent être utilisés à des fins diverses et, en particulier, comme intermédiaires dans la synthèse organique, par exemple, dans la préparation de produits pharmaceutiques, herbicides, insecticides et désinfectants. Les nouveaux isocyanurates de formules (I), (II) et (III) suivant la présente invention peuvent intervenir dans la préparation de polymères.

Le principe de la préparation d'isocyanurates moléculaires, par trimérisation d'isocyanates organiques, en présence de catalyseurs, est connu. Ces préparations, qui ne peuvent conduire qu'à des composés hétérocycliques moléculaires, ne sont en général applicables que pour la préparation d'isocyanurates portant trois substituants identiques.

Il est connu aussi de préparer des sels métalliques d'isocyanurates par réaction d'un isocyanate organique et d'un cyanate métallique. Ainsi, dans le brevet américain n° 3.549.630, on décrit la préparation de sels métalliques d'isocyanurates disubstitués symétriques par réaction, dans un solvant aprotique, d'un isocyanate organique et d'un cyanate métallique; les sels d'isocyanurates obtenus peuvent ensuite être transformés, par réaction avec un halogénure organique, en isocyanurates trisubstitués. Ce procédé présente, comme inconvénients, de nécessiter l'emploi de solvants aprotiques dont la constante diélectrique est supérieure à 15 à 25°C et de ne permettre que la préparation de sels d'isocyanurates disubstitués symétriques à partir desquels il n'est pas possible, par réaction avec un halogénure organique, d'obtenir des isocyanurates trisubstitués dont les trois substituants sont différents.

On connaît également la préparation de sels

métalliques d'isocyanurates à partir de diaryl-1,3-urétidine-2,4 diones et de cyanates métalliques. Ce procédé, décrit dans le brevet américain n° 3.625.964, a l'inconvénient de ne permettre que la préparation de sels d'isocyanurates portant des substituants aromatiques en positions 3 et 5; par ailleurs, la préparation des sels d'isocyanurates portant des substituants différents sur les positions 3 et 5 nécessite la préparation d'urétidine-diones asymétriques qui est, en général, difficile et peu sélective.

Un autre inconvénient de ce procédé est que, en général, il fournit un mélange de sel d'isocyanurate et d'isocyanurate moléculaire. En outre, le procédé n'est réalisé qu'en suspension et le choix des solvants utilisables est limité aux solvants aprotiques dont la constante diélectrique est supérieure à 15 à 25°C.

En plus des préparations d'isocyanurates moléculaires, basées sur la trimérisation des isocyanates ou sur l'action d'un coréactif approprié avec un sel d'isocyanurate, sont décrites dans la littérature les préparations d'isocyanurates portant des substituants différents en position 1,3,5 à partir d'allophanates et d'isocyanates organiques (A. Etienne, G. Lonchambon et J. Roques, C.R. Acad. Sc., Paris, Série C.283, p. 281, 1976) ou à partir de dialcoyl-1,3 aryl-5 bis(diméthylamino)-6,6 triazine 1,3,5 dione-2,4 et d'arylsulfonylisocyanate (R. Richter, H. Ulrich, J. Org. Chem. 41 (21), 3409, 1976).

Ces derniers procédés présentent de nombreux inconvénients : ils utilisent des matières premières coûteuses, ne s'appliquent que pour l'obtention de certains isocyanurates asymétriques et se réalisent souvent à des températures et avec des temps de réaction qui grèvent lourdement l'économie de ces procédés.

Le procédé découvert par la demanderesse ne

présente pas ces inconvénients et permet la préparation, dans des conditions économiques, d'une multitude de triazinates 1,3,5 triones 2,4,6 symétriques ou asymétriques à partir desquelles peuvent être obtenus des isocyanurates ou triazines 1,3,5 triones 2,4,6 moléculaires portant des substituants identiques ou différents en positions 1,3,5.

L'économie du procédé de la présente invention réside dans l'utilisation comme matières premières d'oxadiazines 1,3,5 triones 2,4,6 qui s'obtiennent aisément à partir de réactifs peu coûteux.

Ainsi, la présente invention concerne également un procédé de préparation d'isocyanurates répondant à la formule générale :

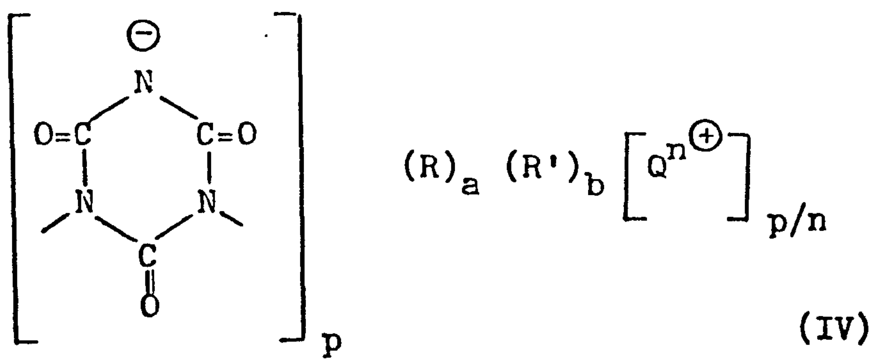

(IV)

dans laquelle

R désigne un radical hydrocarboné éventuellement substitué, monovalent ou polyvalent ;

R' qui peut être identique ou différent de R désigne un radical hydrocarboné éventuellement substitué ou de l'hydrogène ;

Q désigne un métal ou un groupement contenant un nombre n de fonctions ammonium, phosphonium ou arsonium non substituées ou mono-, bi-, tri- ou tétrasubstituées ;

p est un nombre au moins égal à 1 ;

n désigne le nombre d'oxydation du métal ou le nombre de fonctions ammonium, phosphonium ou arsonium présentes dans le groupement Q ;

a est égal à 1 ou à p et b est égal à zéro ou à p, étant

entendu que, si a est égal à 1, b est égal à p et que, si b est égal à zéro, a est égal à p, ce procédé consistant à faire réagir une oxadiazine 1,3,5 trione 2,4,6 répondant à l'une ou l'autre des formules générales suivantes :

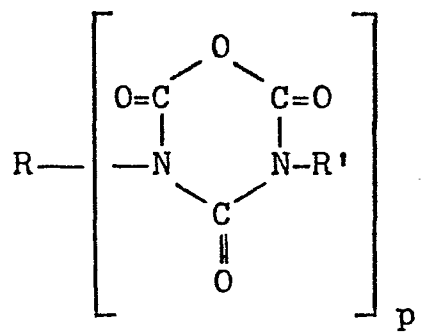

(V)

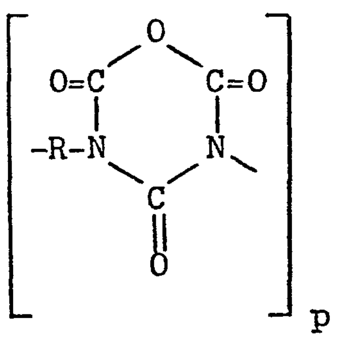

(VI)

dans lesquelles R, R' et p ont les significations données ci-avant, avec un cyanate salin répondant à la formule

$$Q\ (NCO)_n \qquad (VII)$$

dans laquelle Q et n ont les significations données ci-avant.

Le procédé suivant l'invention peut être illustré par les schémas de réactions (A) et (B) suivants :

Schéma A

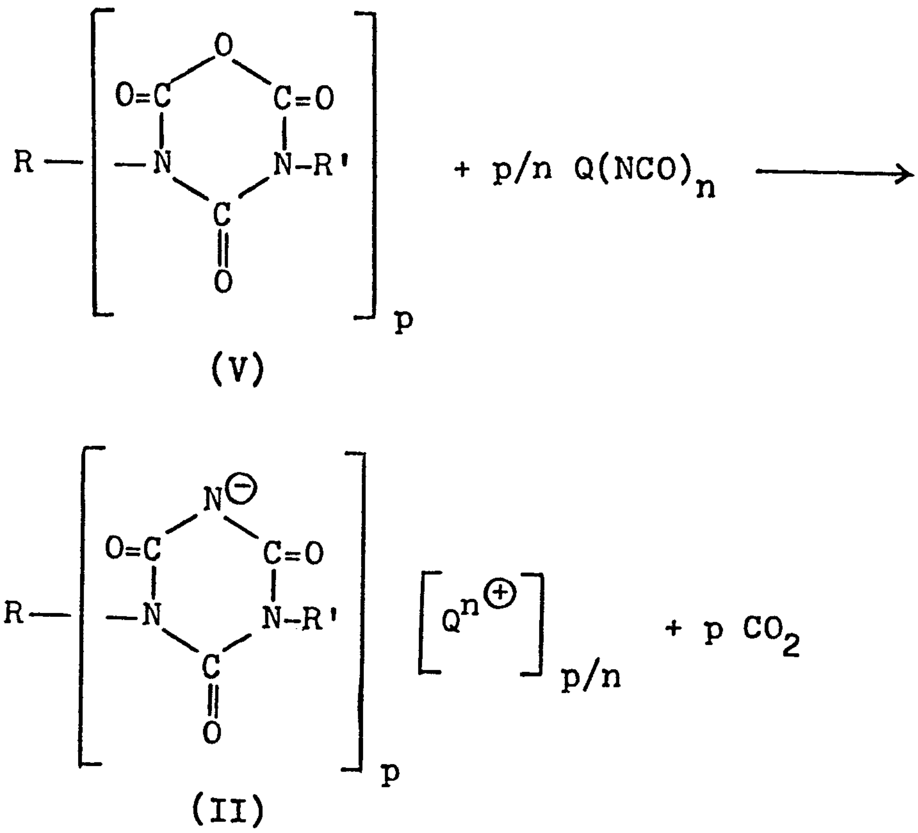

(V)

(II)

Schéma B

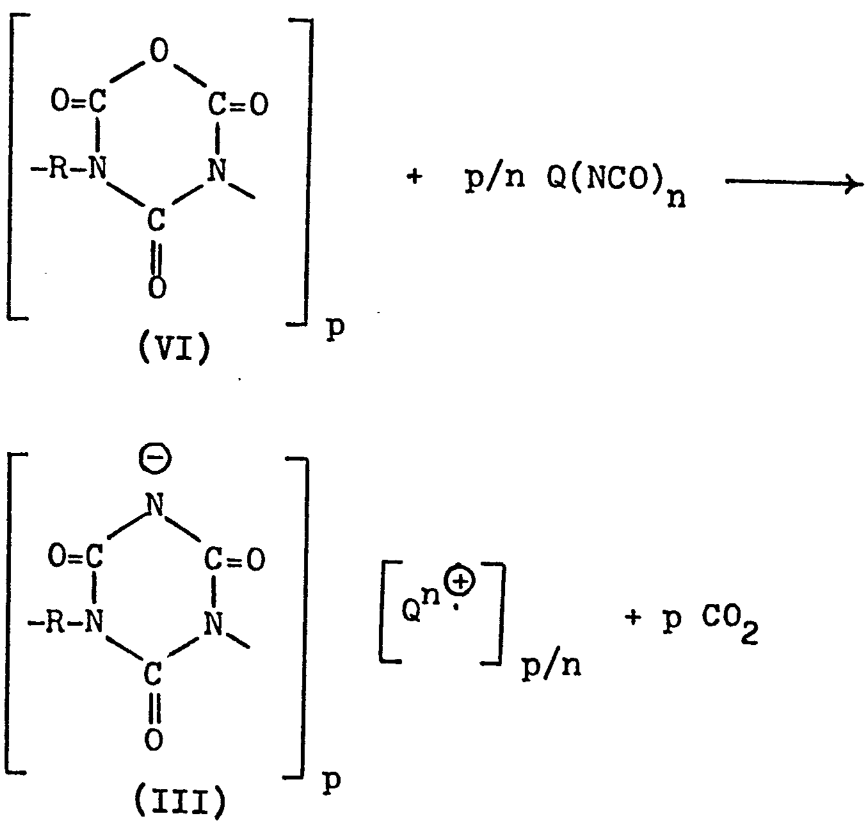

(VI)

(III)

Dans ces schémas de réactions, R, R', Q, p et n ont les significations indiquées plus haut dans la définition des composés répondant à la formule générale (IV).

Comme oxadiazines 1,3,5 triones 2,4,6 de formule (V) ou (VI), on préfère utiliser les oxadiazines 1,3,5 triones 2,4,6 dans lesquelles les atomes d'azote situés en position 3 et 5 du cycle ne sont pas liés directement à un atome de carbone d'un noyau aromatique.

Comme exemples d'oxadiazines 1,3,5 triones 2,4,6 utiles dans le procédé suivant la présente invention, on peut citer :

la diméthyl-3,5 oxadiazine 1,3,5 trione 2,4,6 ;
la méthyl-3 benzyl-5 oxadiazine 1,3,5 trione 2,4,6 ;
la butyl-3 allyl-5 oxadiazine 1,3,5 trione 2,4,6 ;
la benzyl-3 H-5 oxadiazine 1,3,5 trione 2,4,6 ;
la méthyl-3 allyl-5 oxadiazine 1,3,5 trione 2,4,6 ;
la propyl-3 acétyl-5 oxadiazine 1,3,5 trione 2,4,6 ;
la (chloro-9-décyl)-3 cyclohexyl-5 oxadiazine 1,3,5 trione 2,4,6 ;
le bis-1,6-(méthyl-3 oxadiazine 1,3,5 trione 2,4,6)-hexane de formule :

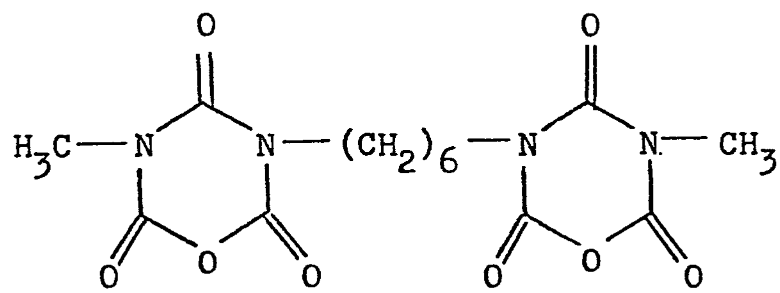

(XVII)

le bis-αα'-(allyl-3 oxadiazine 1,3,5 trione 2,4,6)-p-xylène de formule :

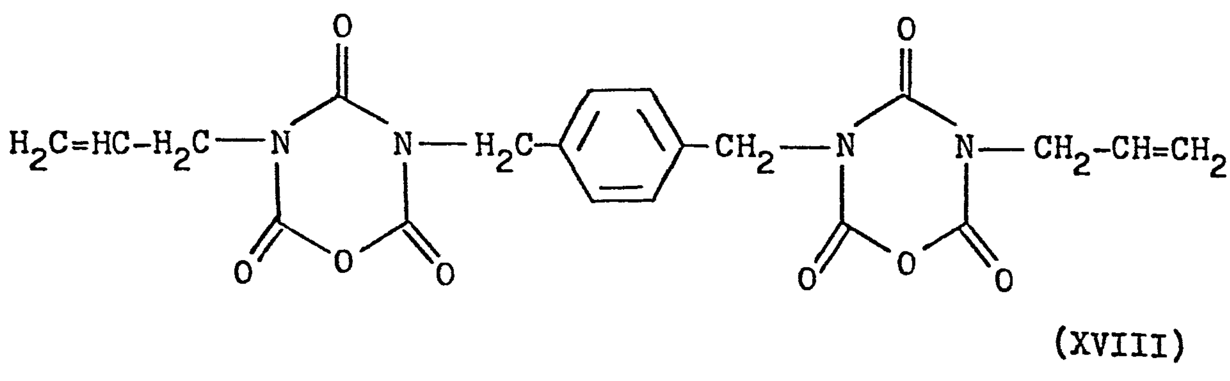

(XVIII)

les poly(méthyl-3 oxadiazines 1,3,5 triones 2,4,6), polymères formés d'un nombre p d'unités récurrentes de formule :

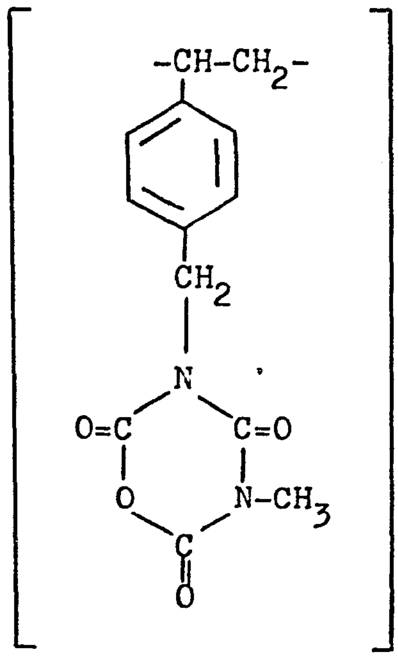

(XIX)

et les polyoxadiazines 1,3,5 triones 2,4,6, polymères formés d'un nombre p d'unités récurrentes de formule :

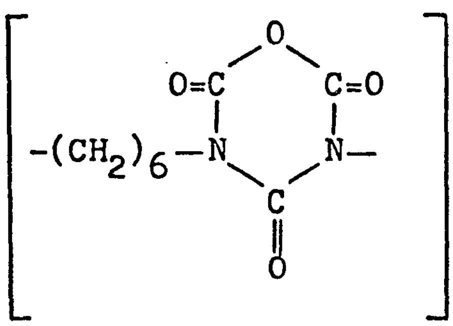

(XX)

ou leurs mélanges.

Les oxadiazines 1,3,5 triones 2,4,6 de formules (V) et (VI) peuvent être introduites telles quelles dans le mélange de réaction ou être produites dans le solvant de la réaction par toute méthode connue. Cette combinaison de la préparation de l'oxadiazine 1,3,5 trione 2,4,6 et de sa transformation en isocyanurate est particulièrement

avantageuse du point de vue économique. La préparation de l'oxadiazine 1,3,5 trione 2,4,6 peut être réalisée, par exemple, suivant un procédé faisant l'objet d'un autre brevet de la demanderesse, à partir d'un isocyanate organique, d'un cyanate salin, d'anhydride carbonique et d'un halogénure ou d'un sulfate organique.

Les cyanates salins de formule (VII), utilisables dans le procédé suivant l'invention, sont des cyanates de métaux mono-, bi- ou trivalents ou des cyanates d'ammonium, de phosphonium ou d'arsonium, non substitués ou mono-, bi-, tri- ou tétrasubstitués. Dans ces cyanates d'ammonium, de phosphonium ou d'arsonium, Q peut être représenté

- soit par la formule générale :

$$(R^{I}\ R^{II}\ R^{III}\ Z)_n\ R^{IV} \qquad \text{(VIII)}$$

dans laquelle Z désigne l'azote, le phosphore et l'arsenic et $R^{I}$, $R^{II}$, $R^{III}$ et $R^{IV}$, qui sont identiques ou différents, désignent de l'hydrogène ou des radicaux alcoyle, aryle, arylalcoyle ou hétérocycliques substitués ou non; $R^{I}$ et $R^{II}$, pris ensemble, peuvent aussi représenter un radical bivalent ou dans laquelle $R^{IV}$ désigne un radical polyvalent et n est le nombre de fonctions ammonium, phosphonium ou arsonium présentes dans le groupement Q du composé de formule (II) ou (III) ;

- soit par la formule générale :

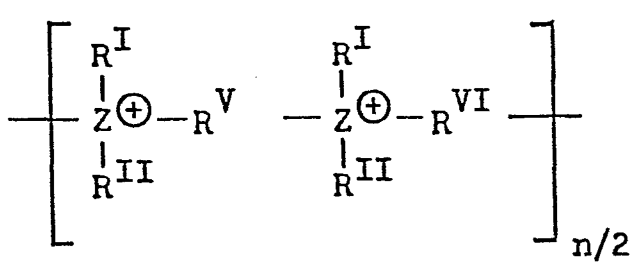

(IX)

dans laquelle Z, $R^{I}$ et $R^{II}$ ont les significations données ci-dessus, $R^{V}$ et $R^{VI}$ sont des radicaux bivalents et n a la signification donnée plus haut.

Le cyanate salin préféré dépend de la réactivité du système, de la température et du solvant éventuel dans lequel la préparation est réalisée.

Dans la plupart des cas, il est avantageux d'utiliser comme cyanate salin un cyanate de métal alcalin ou alcalino-terreux ou des cyanates d'ammonium, phosphonium ou arsonium tétrasubstitués, monovalents ou polyvalents ou leurs mélanges. On accorde la préférence au cyanate de sodium, au cyanate de potassium et aux cyanates d'ammonium tétrasubstitués, mono- ou polyvalents ou à leurs mélanges.

Les cyanates d'ammonium, phosphonium ou arsonium peuvent éventuellement être produits dans le liquide de la préparation, avant ou pendant la préparation, par toute méthode connue.

Des exemples de cyanates d'ammonium, phosphonium et arsonium tétrasubstitués utilisables dans le procédé suivant la présente invention sont le cyanate de tétraméthylammonium, le cyanate de tétraéthylammonium, le cyanate de tétrapropylammonium, le cyanate de tétrabutylammonium, le cyanate de tétraamylammonium, le cyanate de tétrahexylammonium, le cyanate de tricaprylméthylammonium, le cyanate de benzyltriméthylammonium, le cyanate de benzyltriéthylammonium, le cyanate de benzyltributylammonium, le cyanate de benzylisopropyldiméthylammonium, le cyanate de benzyltripropylammonium, le cyanate de phényltriméthylammonium, le cyanate de phényltriéthylammonium, le cyanate de diméthylpipéridinium, le cyanate de méthylpyridinium, le cyanate de méthyltributylphosphonium, le cyanate de tétrabutylphosphonium, le cyanate de tétraphénylphosphonium, le cyanate de benzyltriéthylarsonium, le cyanate de tétrapropylarsonium, le cyanate de tétraphénylarsonium, le cyanate de 1,4-bis(triméthylammonium)-butane, le cyanate de diméthyl-1,4-diaza-1,4-bicyclo-(2,2,2)octane, le cyanate de bis-1,6-(triméthylammonium)-hexane, les cyanates fixés sur une résine anionique du

type AMBERLITE IRA 400 (FLUKA), l'ionène de formule :

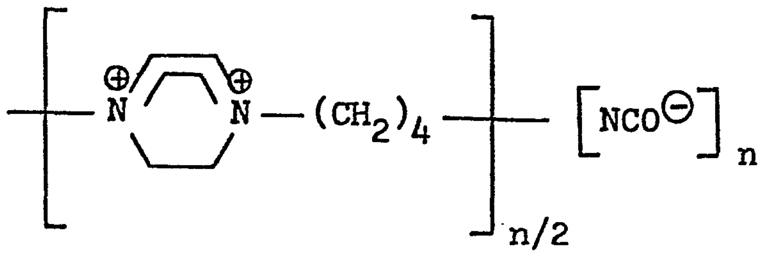

(X),

l'ionène de formule :

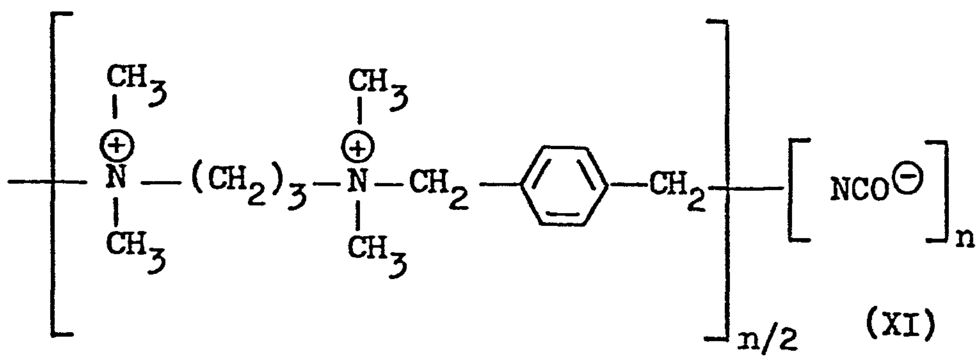

(XI)

où n est compris entre 4 et 40, ou leurs mélanges et leurs dérivés substitués.

L'utilisation de catalyseurs n'est pas indispensable dans le présent procédé qui permet des préparations avec de bons rendements dans des temps assez courts; des catalyseurs tels que $CaCl_2$ ou MgO peuvent cependant être utilisés dans des cas spécifiques.

Il est avantageux que les substances qui sont alimentées à l'état de matière solide soient sous la forme la plus divisée possible.

Les conditions de réaction, telles que la température, la pression et le solvant dans lequel se déroulent les réactions ainsi que les rapports quantitatifs des substances à mettre en oeuvre, dépendent dans une certaine mesure l'une de l'autre et se choisissent,

le cas échéant, selon le type de substance utilisée et la nature du produit désiré.

Le rapport quantitatif du cyanate salin à l'oxadiazine 1,3,5 trione 2,4,6 peut être choisi dans de larges limites, ce rapport pouvant être aussi bien stoechiométrique que supérieur ou inférieur à la stoechiométrie. En général, on utilise, par équivalent de cyanate salin, 0,5 à 2 équivalents d'oxadiazine 1,3,5 trione 2,4,6 et, de préférence, 0,8 à 1,2 équivalent d'oxadiazine 1,3,5 trione 2,4,6 par équivalent de cyanate salin.

Le procédé de la présente invention, bien que réalisable en l'absence de solvant, est effectué de préférence dans un solvant organique inerte n'interférant pas avec la réaction.

On peut utiliser aussi bien des solvants organiques apolaires ou peu polaires que des solvants organiques polaires. Comme solvants utilisables dans la présente invention, on peut citer, par exemple, le tétrachlorure de carbone, le chloroforme, le dichlorométhane, le 1,2-dichloréthane, le 1,1-dichloréthane, le mélange d'isomères cis et trans du 1,2-dichloréthylène, l'o-dichlorobenzène, le dioxanne, l'acétate d'éthyle, le diméthoxyéthane, le tétrahydrofuranne, le toluène, l'α-méthylnaphtalène, l'acétone, la méthyléthylcétone, l'acétonitrile, le propionitrile, le benzonitrile, le cyanure de benzyle, le nitrobenzène, le nitrotoluène, le diméthylformamide, le diméthylacétamide, la tétraméthylurée, la N-méthylpyrrolidone, l'hexaméthylphosphotriamide, le diméthylsulfoxyde ainsi que leurs mélanges. Conviennent tout particulièrement les hydrocarbures chlorés, les éthers linéaires ou cycliques, les cétones et les nitriles.

La proportion de solvant dépend, dans une large mesure, du type de solvant et du type de rapport quantitatif des autres substances. En général, pour chaque

partie en poids de réactifs, on peut utiliser jusque 50 parties en poids de solvant. Dans la plupart des cas, il est avantageux d'utiliser 0,2 à 25 parties en poids de solvant, de préférence de 1 à 10 parties en poids de solvant, pour une partie en poids de réactifs.

La préparation des isocyanurates de formules (I) à (IV) peut être réalisée suivant l'invention à des températures comprises entre environ -80°C et +250°C. Dans la plupart des cas, on peut utiliser des températures comprises entre -20°C et 150°C et, plus spécialement, des températures comprises entre 0°C et 70°C.

Il est généralement avantageux de travailler à des pressions qui sont de l'ordre de 0,6 à 4 bars, en particulier, à la pression atmosphérique; on peut cependant également recourir à des pressions plus élevées. Cependant, si l'on veut utiliser des appareillages simples, il est avantageux de travailler à des pressions qui ne sont pas sensiblement différentes de la pression ordinaire.

Le temps de réaction peut varier dans une large mesure suivant la réactivité des réactifs, la température, la nature du solvant et la présence éventuelle de catalyseurs. En général, le temps de réaction est compris entre 0,5 sec. et 24 heures et le plus souvent entre 1 sec. et 10 heures.

Les rendements en isocyanurates des formules (I) à (IV) sont en général élevés et peuvent être supérieurs à 90 % après 1 minute de réaction à 25°C.

Les isocyanurates de formules (I) à (IV) obtenus par le procédé susdécrit suivant l'invention peuvent être transformés, par des méthodes connues, en triazines 1,3,5 triones 2,4,6 moléculaires mono-, bi- ou trisubstituées dont les substituants dans les positions 1,3 et 5 peuvent être identiques ou différents.

La présente invention couvre donc également la préparation des triazines 1,3,5 triones 2,4,6 moléculaires répondant aux formules générales suivantes :

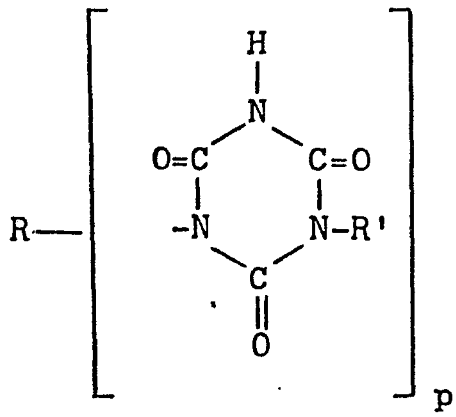

(XII)

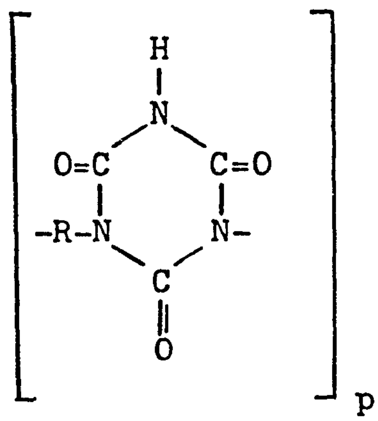

(XIII)

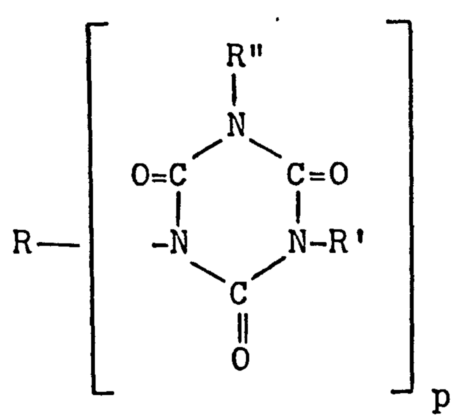

(XIV)

ou

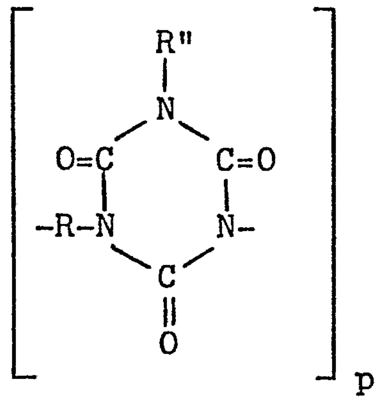

(XV)

Dans ces formules (XII) à (XV), R, R' et p ont les mêmes significations que dans les composés de formules générales (I) à (IV), tandis que R" représente un radical hydrocarboné, substitué ou non, identique ou différent de R et de R'.

Les triazines 1,3,5 triones 2,4,6 mono- ou bisubstituées symétriques ou asymétriques répondant aux formules (XII) et (XIII) peuvent être préparées, conformément à l'invention, par réaction d'un acide avec un isocyanurate de formule (I) à (IV).

Comme acide, on peut utiliser tout acide organique ou minéral susceptible de céder un proton aux triazinates 1,3,5 triones 2,4,6 qui possèdent des propriétés basiques, par exemple un acide choisi parmi l'acide chlorhydrique, l'acide sulfurique, l'acide acétique, l'acide benzoïque, l'acide formique et l'acide oxalique. La neutralisation peut être réalisée indifféremment dans le solvant organique de la préparation, dans l'eau, dans un autre solvant ou dans un mélange de solvants.

Quant aux triazines 1,3,5 triones 2,4,6 trisubstituées symétriques ou asymétriques répondant aux formules (XIV) et (XV), elles peuvent être préparées, conformément à la présente invention, par réaction d'un

isocyanurate de formule (I) à (IV) avec un halogénure ou sulfate organique de formule

R"Y (XVI)

dans laquelle R" représente un radical hydrocarboné, substitué ou non, identique ou différent de R et de R', tandis que Y désigne un halogène ou un groupement sulfate.

Comme exemples d'halogénures et de sulfates organiques utilisables dans la présente invention, on peut citer le sulfate de diméthyle, le sulfate de diéthyle, le chlorure de benzyle, le chlorure d'allyle, le 1-chloro-but-2-ène, le chlorure de cyclohexyle, le chlorure de méthyle, l'iodure de méthyle, l'α-chloro-p-nitrotoluène, le 1-chlorohexane, l'α-chloro-p-trifluorométhyltoluène, le chlorure d'acétyle, le chlorure de benzoyle, le chlorure de p-toluènesulfonyle, le chlorure d'oxalyle, les chlorures de phtalyle, le 1,4-dichlorobutène, l'α,α'-dichloro-m-xylène, l'α,α'-dichloro-p-xylène, le 1,6-dichlorohexane ou leur combinaison et leurs dérivés substitués.

L'invention sera décrite plus en détail à l'aide des exemples qui suivent, destinés à illustrer l'invention sans la limiter.

Les exemples décrits ont été réalisés en discontinu dans des réacteurs du type Grignard; il est évident que des réacteurs opérant en continu peuvent aussi être utilisés.

Les produits obtenus ont été identifiés par détermination de leurs compositions élémentaires, titrage potentiométrique, analyse par spectroscopie I.R., de résonance magnétique nucléaire du $^{13}C$ et spectroscopie de masse.

EXEMPLE 1

L'appareil est un réacteur en verre de 0,5 l muni de quatre ouvertures, équipé d'un agitateur mécanique, d'un condenseur à reflux, d'un thermomètre et

d'un dispositif permettant l'introduction d'un liquide. La température est réglée à l'aide d'un thermostat.

On introduit dans le réacteur 34,0 g de cyanate de tétraamylammonium (0,1 mole), 15,8 g de diméthyl-3,5 oxadiazine 1,3,5 trione 2,4,6 (0,1 mole) et 250 ml de tétrahydrofuranne anhydre; le mélange réactionnel initialement hétérogène est laissé sous agitation pendant 30 minutes à 25°C; on observe un dégagement d'anhydride carbonique.

Par évaporation du solvant sous vide, on obtient 45 g de diméthyl-3,5 isocyanurate de tétraamylammonium (rendement 99 %).

La pureté du produit obtenu par simple évaporation du solvant est très élevée; le lavage du solide par le tétrachlorure de carbone pour éliminer le triméthyl 1,3,5 isocyanurate éventuellement formé et la cristallisation dans un mélange de tétrahydrofuranne et d'éther de pétrole ne modifient pas les caractéristiques du produit.

EXEMPLE 2

On opère dans les conditions décrites dans l'exemple 1. On utilise 23,4 g de méthyl-3 benzyl-5 oxadiazine 1,3,5 trione 2,4,6, 22,8 g de cyanate de tétrapropylammonium (0,1 mole) et 250 ml de dichlorométhane. Le mélange réactionnel homogène est laissé sous agitation pendant 5 minutes; ensuite le solvant est évaporé. On obtient 40,1 g de méthyl-3 benzyl-5 isocyanurate de tétrapropylammonium (rendement 96 %).

EXEMPLE 3

On opère comme dans l'exemple 2 et à la solution de méthyl-3 benzyl-5 isocyanurate de tétrapropylammonium, on ajoute 7,9 g de chlorure d'acétyle (0,1 mole); après 30 minutes de réaction, on obtient la méthyl-3 benzyl-5 acétyl-1 triazine 1,3,5 trione 2,4,6 avec un rendement de 95 %.

EXEMPLE 4

On utilise le réacteur décrit dans l'exemple 1. A 18,5 g de 1,6-bis(méthyl-3 oxadiazine 1,3,5 trione 2,4,6)hexane (0,05 mole) dans 250 ml de dichlorométhane sec, on ajoute progressivement, en 10 minutes, 28,4 g de cyanate de tétrabutylammonium solide (0,1 mole). Après 20 minutes de réaction sous agitation à 25°C, on évapore le solvant; on obtient 39,5 g de 1,6-bis(méthyl-3 triazinate 1,3,5 trione 2,4,6)hexane de tétrabutylammonium (rendement 93 %).

EXEMPLE 5

Dans le réacteur décrit dans l'exemple 1, on introduit 17,2 g de méthyl-3 éthyl-5 oxadiazine 1,3,5 trione 2,4,6 et 250 ml d'acétonitrile anhydre; on y ajoute 22,8 g de cyanate de tétrapropylammonium (0,1 mole). Après 5 minutes de réaction à 25°C, on récupère, après évaporation du solvant, 34,2 g de méthyl-3 éthyl-5 isocyanurate de tétrapropylammonium (rendement 96 %).

EXEMPLE 6

Dans le réacteur décrit dans l'exemple 1, on introduit 7,9 g de diméthyl-3,5 oxadiazine 1,3,5 trione 2,4,6 (0,1 mole), 250 ml de tétrahydrofuranne anhydre et 21,2 g de cyanate de tétraphénylarsonium; le mélange réactionnel initialement hétérogène est laissé sous agitation à 25°C jusqu'au moment où le dégagement de $CO_2$ s'arrête. On isole 22,3 g (rendement 84 %) de diméthyl-3,5 isocyanurate de tétraphénylarsonium.

EXEMPLE 7

Dans le réacteur décrit dans l'exemple 1, on introduit 0,1 mole de méthyl-3 éthyl-5 oxadiazine 1,3,5 trione 2,4,6 et 250 ml d'acétonitrile; après dissolution du réactif, on introduit sous agitation, à 20°C, 0,1 mole de cyanate de tétrabutylphosphonium. Après la fin du dégagement de $CO_2$ (10 minutes), on isole le méthyl-3 éthyl-5 isocyanurate de tétrabutylphosphonium (rendement 97 %).

EXEMPLE 8

Dans le réacteur décrit dans l'exemple 1, on introduit 0,08 mole de cyanate de potassium finement divisé et 250 ml de diméthylsulfoxyde; à ce mélange sous agitation à 25°C, on ajoute 0,08 mole de diméthyl-3,5 oxadiazine 1,3,5 trione 2,4,6. Le dégagement de $CO_2$ cesse après 5 minutes; le produit peu soluble est isolé par filtration et lavé à l'éther. On isole 6 g (rendement 39 %) de diméthyl-3,5 isocyanurate de potassium.

EXEMPLE 9

La polymérisation de l'hexaméthylènediisocyanate avec l'anhydride carbonique, en présence de tributylphosphine, a été réalisée dans les conditions utilisées par K.H. Slotta et R. Tschesche [Ber., 60, 295 (1927)] pour la préparation de monooxadiazines.

Du polymère obtenu, on a isolé la fraction soluble dans le tétrahydrofuranne; la polyoxadiazine obtenue tombant dans la formule (VI) a la composition suivante :

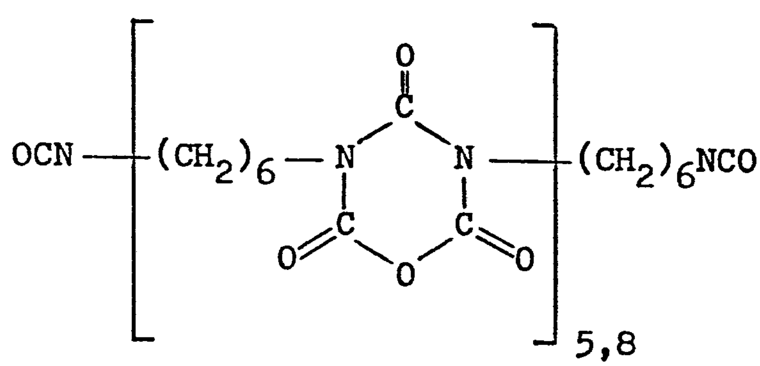

(XXI)

On introduit dans le réacteur décrit dans l'exemple 1, sous agitation et à 25°C, 15,7 g de cette polyoxadiazine, 250 ml de tétrahydrofuranne et 15 g de cyanate de tétrapropylammonium.

Le produit de la réaction précipite en moins de 5 minutes. Après filtration, on isole 22,5 g (rendement 81 %) d'un polyisocyanurate de tétrapropylammonium, tombant dans la formule (III), possédant en bout de chaîne des fonctions isocyanates.

EXEMPLE 10

On introduit dans le réacteur décrit dans l'exemple 1, 10 g de 1,6-bis(méthyl-3 triazinate 1,3,5 trione 2,4,6)hexane de tétrapropylammonium préparé comme dans l'exemple 4 et 100 ml de dichlorométhane. On introduit ensuite sous agitation et à température normale, une solution d'acide chlorhydrique dans le même solvant jusqu'à neutralisation totale des fonctions basiques. Le produit de la réaction est le 1,6-bis(méthyl-3 triazine 1,3,5 trione 2,4,6)hexane tombant dans la formule (XII). La réaction est quantitative.

EXEMPLE 11

On ajoute à une solution de 10 g de 1,5-bis(allyl-3 triazinate 1,3,5 trione 2,4,6)hexane de tétrapropylammonium préparé comme dans l'exemple 4 et 100 ml de dichlorométhane anhydre, sous agitation et à 25°C, 0,03 mole de diméthylsulfate. La réaction est poursuivie pendant 30 minutes à température normale. On isole 5,2 g (rendement 95 %) de 1,6-bis(allyl-3 méthyl-5 triazine 1,3,5 trione 2,4,6)hexane, tombant dans la formule (XIV).

EXEMPLE 12

On introduit dans le réacteur décrit dans l'exemple 1, 10 g de polyisocyanurate de tétrapropylammonium préparé comme dans l'exemple 9 et 100 ml de méthanol. On ajoute à la suspension, sous agitation et à température normale, une solution 0,5 M de HCl dans le méthanol jusqu'à neutralisation de toutes les fonctions basiques. L'analyse du produit obtenu montre qu'en plus de la neutralisation des fonctions triazinates, la transformation convertit également les fonctions isocyanates terminales en uréthanne de méthyle. La structure du produit final est la suivante :

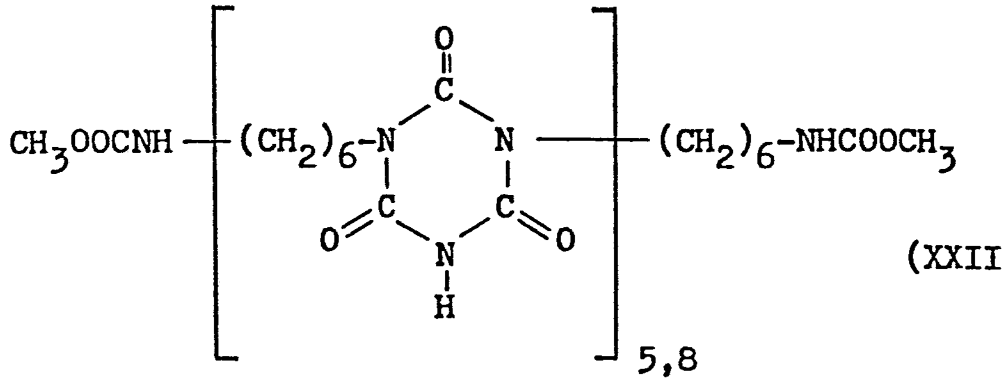

(XXII)

tombant dans la formule (XIII).

## R E V E N D I C A T I O N S

1. A titre de nouveaux composés, les isocyanurates répondant à la formule générale suivante :

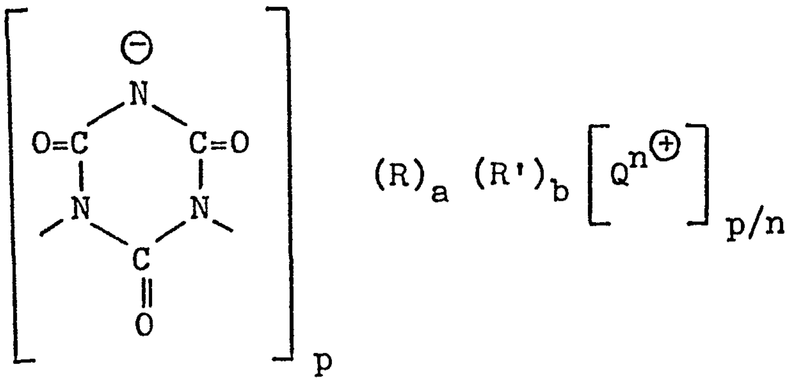

(I)

dans laquelle

R désigne un radical hydrocarboné éventuellement substitué, monovalent ou polyvalent ;

R' qui est différent de R désigne un radical hydrocarboné éventuellement substitué ou de l'hydrogène ;

Q désigne un groupement contenant un nombre n de fonctions ammonium, phosphonium ou arsonium tétrasubstituées ;

p est un nombre au moins égal à 1 ;

a est égal à 1 ou à p et b est égal à zéro ou à p, étant entendu que, si a est égal à 1, b est égal à p et que, si b est égal à zéro, a est égal à p.

2. Isocyanurates suivant la revendication 1, caractérisés en ce qu'ils répondent aux formules générales suivantes :

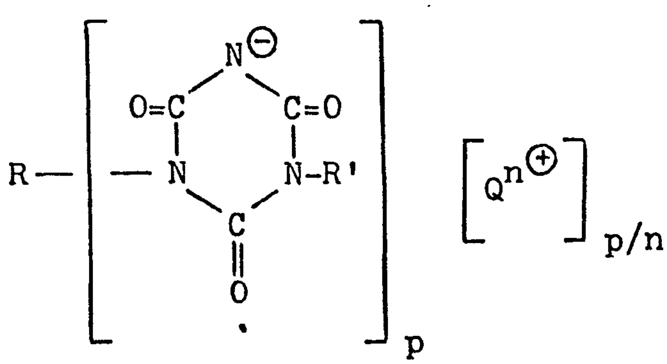

(II)

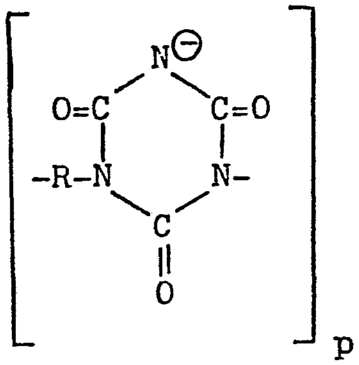 $\left[Q^{n\oplus}\right]_{p/n}$ (III)

dans lesquelles

Q désigne un groupement contenant un nombre n de fonctions ammonium, phosphonium ou arsonium tétrasubstituées ;

R désigne un radical hydrocarboné éventuellement substitué, monovalent ou polyvalent ;

R' désigne un radical hydrocarboné éventuellement substitué, différent de R, ou de l'hydrogène ;

p est un nombre égal ou supérieur à 1 ;

n désigne le nombre de fonctions ammonium, phosphonium ou arsonium présentes dans le groupement Q.

3. Procédé de préparation d'isocyanurates répondant à la formule générale :

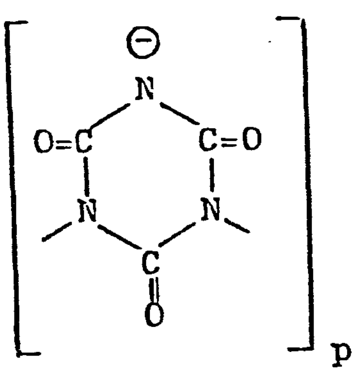 $(R)_a\ (R')_b \left[Q^{n\oplus}\right]_{p/n}$ (IV)

dans laquelle

R désigne un radical hydrocarboné éventuellement substitué, monovalent ou polyvalent ;

R' qui peut être identique ou différent de R désigne

un radical hydrocarboné éventuellement substitué ou de l'hydrogène ;

Q désigne un métal ou un groupement contenant un nombre n de fonctions ammonium, phosphonium ou arsonium non substituées ou mono-, bi-, tri- ou tétrasubstituées ;

p est un nombre au moins égal à 1 ;

n désigne le nombre d'oxydation du métal ou le nombre de fonctions ammonium, phosphonium ou arsonium présentes dans le groupement Q ;

a est égal à 1 ou à p et b est égal à zéro ou à p, étant entendu que, si a est égal à 1, b est égal à p et que, si b est égal à zéro, a est égal à p,

ce procédé consistant à faire réagir une oxadiazine 1,3,5 trione 2,4,6 répondant à l'une ou l'autre des formules générales suivantes :

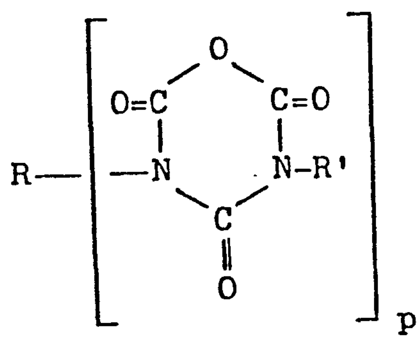

(V)

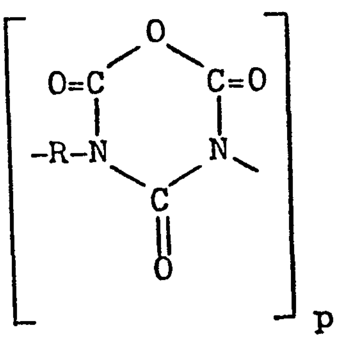

(VI)

dans lesquelles R, R' et p ont les significations données ci-avant, avec un cyanate salin répondant à la formule

Q $(NCO)_n$ (VII)

dans laquelle Q et n ont les significations données ci-avant.

4. Procédé suivant la revendication 3, caractérisé en ce qu'on utilise comme oxadiazines 1,3,5 triones 2,4,6 de formule (V) ou (VI) les oxadiazines 1,3,5 triones 2,4,6 dans lesquelles les atomes d'azote situés en position 3 et 5 du cycle ne sont pas liés directement à un atome de carbone d'un noyau aromatique.

5. Procédé suivant la revendication 4, caractérisé en ce qu'on utilise comme oxadiazines 1,3,5 triones 2,4,6 de formule (V) ou (VI) la diméthyl-3,5 oxadiazine 1,3,5 trione 2,4,6; la méthyl-3 benzyl-5 oxadiazine 1,3,5 trione 2,4,6; la butyl-3 allyl-5 oxadiazine 1,3,5 trione 2,4,6; la benzyl-3 H-5 oxadiazine 1,3,5 trione 2,4,6; la méthyl-3 allyl-5 oxadiazine 1,3,5 trione 2,4,6; la propyl-3 acétyl-5 oxadiazine 1,3,5 trione 2,4,6; la (chloro-9-décyl)-3 cyclohexyl-5 oxadiazine 1,3,5 trione 2,4,6; le bis-1,6-(méthyl-3 oxadiazine 1,3,5 trione 2,4,6)-hexane de formule :

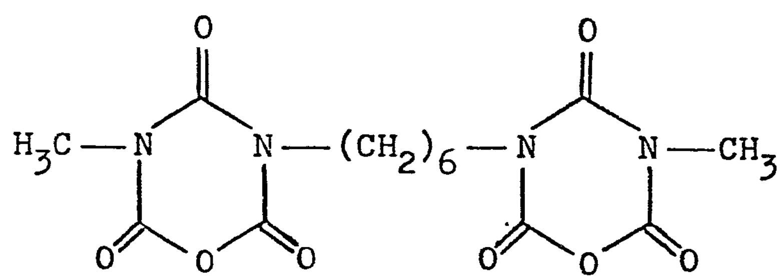

(XVII)

le bis-αα'-(allyl-3 oxadiazine 1,3,5 trione 2,4,6)-p-xylène de formule :

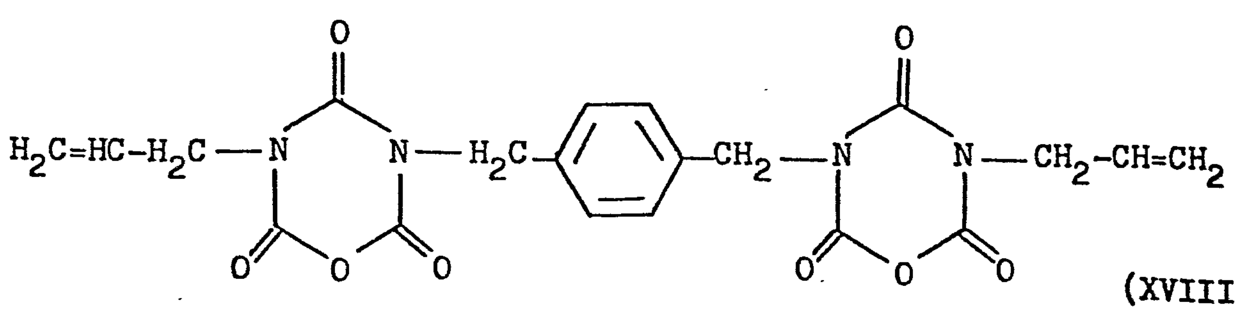

(XVIII)

les poly(méthyl-3 oxadiazines 1,3,5 triones 2,4,6), polymères formés d'un nombre p d'unités récurrentes de formule :

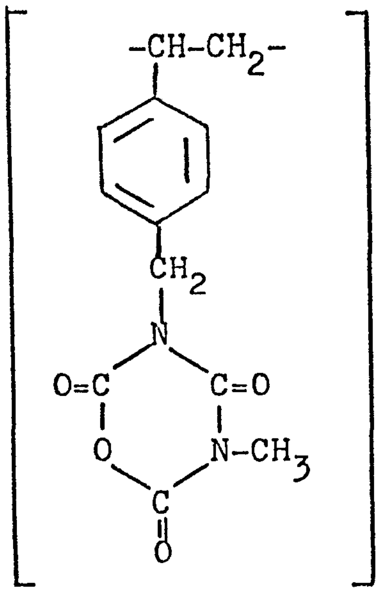

(XIX)

et les polyoxadiazines 1,3,5 triones 2,4,6, polymères formés d'un nombre p d'unités récurrentes de formule :

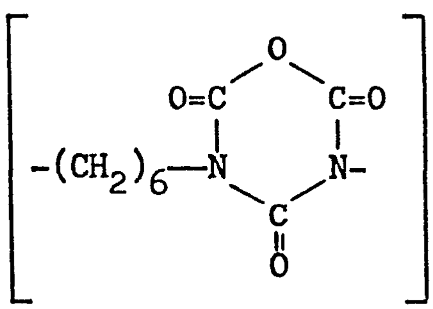

(XX)

ou leurs mélanges.

6. Procédé suivant l'une quelconque des revendications 3 à 5, caractérisé en ce qu'on utilise, comme cyanate salin, un cyanate d'un métal mono-, bi- ou trivalent ou un cyanate d'ammonium, de phosphonium ou d'arsonium non substitué ou mono-, bi-, tri- ou tétrasubstitué.

7. Procédé suivant la revendication 6, caractérisé en ce qu'on utilise, comme cyanate salin,

un cyanate répondant à la formule

$$Q\ (NCO)_n \qquad (VII)$$

dans laquelle n a la signification donnée plus haut et Q désigne un radical de formule

$$(R^{I}\ R^{II}\ R^{III}\ Z)_n\ R^{IV} \qquad (VIII)$$

dans laquelle Z désigne l'azote, le phosphore ou l'arsenic et $R^{I}$, $R^{II}$, $R^{III}$ et $R^{IV}$, qui sont identiques ou différents, désignent de l'hydrogène ou des radicaux alcoyle, aryle, arylalcoyle ou hétérocycliques substitués ou non; $R^{I}$ et $R^{II}$, pris ensemble, peuvent aussi représenter un radical bivalent ou dans laquelle $R^{IV}$ désigne un radical polyvalent et n est le nombre de fonctions ammonium, phosphonium ou arsonium présentes dans le groupement Q du composé de formule (II) ou (III).

8. Procédé suivant la revendication 6, caractérisé en ce qu'on utilise comme cyanate salin un cyanate répondant à la formule

$$Q\ (NCO)_n \qquad (VII)$$

dans laquelle n a la signification donnée plus haut et Q désigne un radical de formule

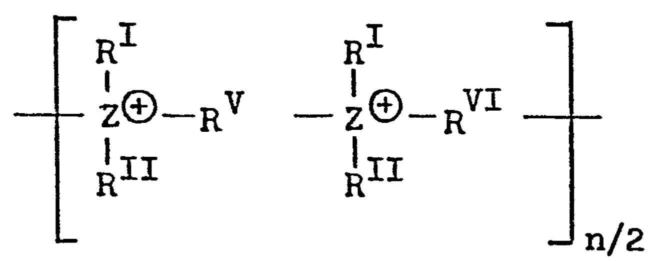

(IX)

dans laquelle Z, $R^{I}$ et $R^{II}$ ont les significations données ci-dessus, $R^{V}$ et $R^{VI}$ sont des radicaux bivalents et n a la signification donnée plus haut.

9. Procédé suivant la revendication 6, caractérisé en ce qu'on utilise comme cyanate salin un cyanate de métal alcalin ou alcalino-terreux.

10. Procédé suivant la revendication 9, caractérisé en ce qu'on utilise comme cyanate salin du cyanate de sodium ou de potassium.

11. Procédé suivant la revendication 6, caractérisé en ce qu'on utilise comme cyanate salin le cyanate de tétraméthylammonium, le cyanate de tétraéthylammonium, le cyanate de tétrapropylammonium, le cyanate de tétrabutylammonium, le cyanate de tétraamylammonium, le cyanate de tétrahexylammonium, le cyanate de tricaprylméthylammonium, le cyanate de benzyltriméthylammonium, le cyanate de benzyltriéthylammonium, le cyanate de benzyltributylammonium, le cyanate de benzylisopropyldiméthylammonium, le cyanate de benzyltripropylammonium, le cyanate de phényltriméthylammonium, le cyanate de phényltriéthylammonium, le cyanate de diméthylpipéridinium, le cyanate de méthylpyridinium, le cyanate de méthyltributylphosphonium, le cyanate de tétrabutylphosphonium, le cyanate de tétraphénylphosphonium, le cyanate de benzyltriéthylarsonium, le cyanate de tétrapropylarsonium, le cyanate de tétraphénylarsonium, le cyanate de 1,4-bis(triméthylammonium)-butane, le cyanate de diméthyl-1,4-diaza-1,4-bicyclo(2,2,2)-octane, le cyanate de bis-1,6-(triméthylammonium)hexane, les cyanates fixés sur une résine anionique du type AMBERLITE IRA 400 (FLUKA),
l'ionène de formule :

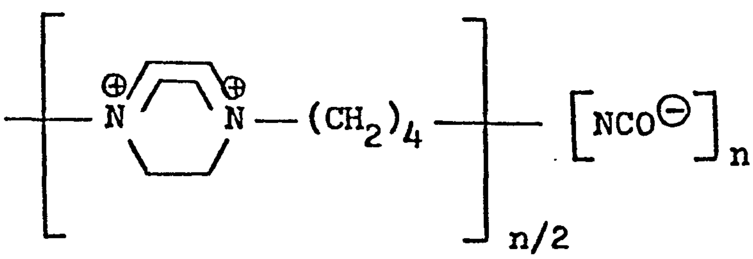

(X) ,

l'ionène de formule :

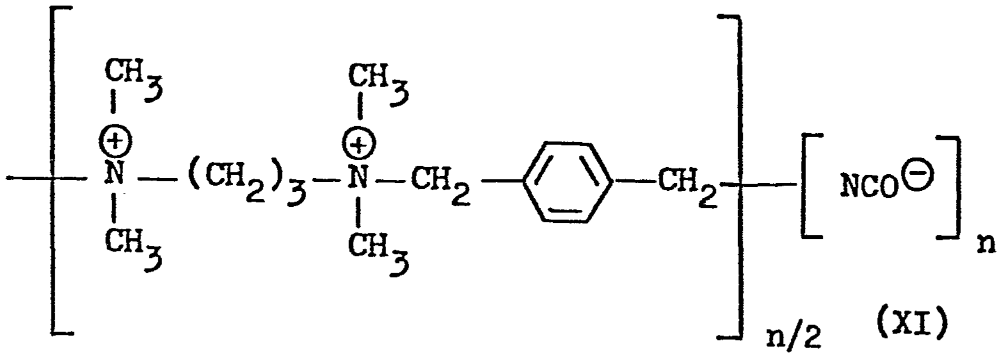

(XI)

où n est compris entre 4 et 40, ou leurs mélanges et leurs dérivés substitués.

12. Procédé suivant l'une quelconque des revendications 3 à 11, caractérisé en ce qu'on utilise un catalyseur.

13. Procédé suivant l'une quelconque des revendications 3 à 12, caractérisé en ce qu'on utilise, par équivalent de cyanate salin, environ 0,5 à 2 équivalents d'oxadiazine 1,3,5 trione 2,4,6 de formule (V) ou (VI).

14. Procédé suivant la revendication 13, caractérisé en ce qu'on utilise, par équivalent de cyanate salin, environ 0,8 à 1,2 équivalent d'oxadiazine 1,3,5 trione 2,4,6 de formule (V) ou (VI).

15. Procédé suivant l'une quelconque des revendications 3 à 14, caractérisé en ce qu'on opère dans un solvant organique inerte.

16. Procédé suivant la revendication 15, caractérisé en ce qu'on utilise comme solvant le tétrachlorure de carbone, le chloroforme, le dichlorométhane, le 1,2-dichloréthane, le 1,1-dichloréthane, le mélange d'isomères cis et trans du 1,2-dichloréthylène, l'o-dichlorobenzène, le dioxanne, l'acétate d'éthyle, le diméthoxyéthane, le tétrahydrofuranne, le toluène, l'α-méthylnaphtalène, l'acétone, la méthyléthylcétone, l'acétonitrile, le propionitrile, le benzonitrile, le

cyanure de benzyle, le nitrobenzène, le nitrotoluène, le diméthylformamide, le diméthylacétamide, la tétraméthylurée, la N-méthylpyrrolidone, l'hexaméthylphosphotriamide, le diméthylsulfoxyde ainsi que leurs mélanges.

17. Procédé suivant l'une quelconque des revendications 3 à 16, caractérisé en ce qu'on opère à une température comprise entre environ -80°C et +250°C.

18. Procédé suivant la revendication 17, caractérisé en ce qu'on opère à une température comprise entre environ -20°C et +150°C.

19. Procédé suivant la revendication 18, caractérisé en ce qu'on opère à une température comprise entre environ 0°C et 70°C.

20. Procédé de préparation d'isocyanurates moléculaires mono- ou bisubstitués répondant aux formules générales suivantes :

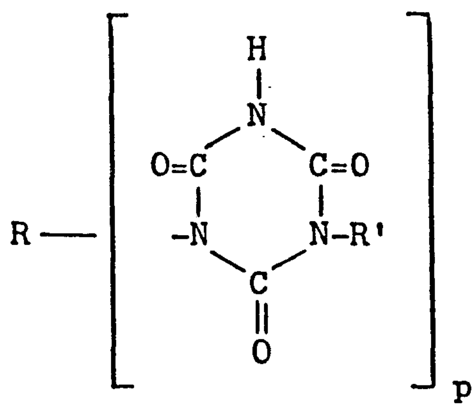

(XII)

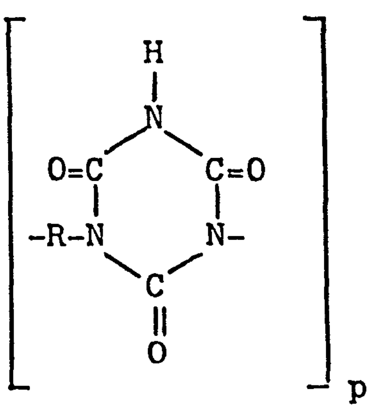

(XIII)

dans lesquelles R, R' et p ont les mêmes significations que dans les composés de formules générales (I) à (IV), caractérisé en ce qu'on fait réagir un acide avec un isocyanurate de formules (I) à (IV).

21. Procédé de préparation d'isocyanurates moléculaires trisubstitués symétriques ou asymétriques répondant aux formules (XIV) et (XV) :

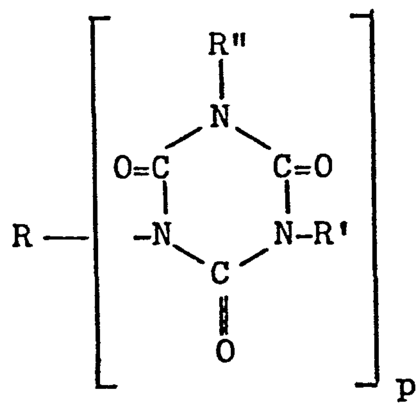

(XIV)

ou

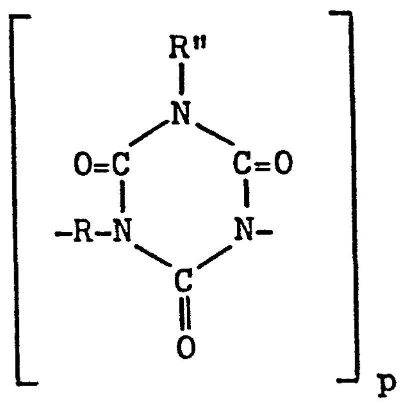

(XV)

dans lesquelles R, R' et p ont les mêmes significations que dans les composés de formules générales (I) à (IV), tandis que R" représente un radical hydrocarboné, substitué ou non, identique ou différent de R et de R', caractérisé en ce qu'on fait réagir un isocyanurate de formules générales (I) à (IV), avec un halogénure ou un sulfate organique de formule :

R"Y (XVI)

dans laquelle R" représente un radical hydrocarboné, substitué ou non, identique ou différent de R et de R', tandis que Y désigne un halogène ou un groupement sulfate.